(19) Europäisches Patentamt
European Patent Office
Office européen des brevets

(11) EP 2 698 383 B1

(12) **EUROPEAN PATENT SPECIFICATION**

(45) Date of publication and mention of the grant of the patent:
**25.11.2020 Bulletin 2020/48**

(51) Int Cl.:
***C07K 16/44*** (2006.01) ***G01N 33/94*** (2006.01)

(21) Application number: **12180486.8**

(22) Date of filing: **14.08.2012**

(54) **Detection of synthetic Cannabinoids**

Nachweis von synthetischen Cannabionoiden

Détection de cannabinoïdes synthétiques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**19.02.2014 Bulletin 2014/08**

(73) Proprietor: **Randox Laboratories Ltd.**
**Crumlin, County Antrim BT29 4QY (GB)**

(72) Inventors:
- **Fitzgerald, Stephen Peter**
  **Crumlin, Antrim BT29 4QY (GB)**
- **Innocenzi, Paul John**
  **Crumlin, Antrim BT29 4QY (GB)**
- **McConnell, Ivan Robert**
  **Crumlin, Antrim BT29 4QY (GB)**
- **Lowry, Philip Andrew**
  **Crumlin, Antrim BT29 4QY (GB)**
- **Benchikh, Elouard**
  **Crumlin, Antrim BT29 4QY (GB)**

(74) Representative: **Gill Jennings & Every LLP**
**The Broadgate Tower**
**20 Primrose Street**
**London EC2A 2ES (GB)**

(56) References cited:
**EP-A1- 0 736 529** **EP-A1- 2 487 155**

- **Anonymous: "Randox Product List 2012", , 1 January 2012 (2012-01-01), XP55038674, Retrieved from the Internet: URL:http://www.randox.com/brochures/PDF Brochure/LT110.pdf [retrieved on 2012-09-19]**
- **WATANABE KAZUHITO ET AL: "CROSS-REACTIVITY OF VARIOUS TETRAHYDROCANNABINOL METABOLITES WITH A MONOCLONAL ANTIBODY AGAINST TETRAHYDROCANNABINOLIC ACID", JOURNAL OF HEALTH SCIENCE - EISEI KAGAKU, PHARMACEUTICAL SOCIETY OF JAPAN, JP, vol. 46, no. 4, 1 January 2000 (2000-01-01), pages 310-313, XP008070074, ISSN: 1344-9702**
- **HIROYUKI TANAKA ET AL: "Monoclonal antibody against tetrahydrocannabinolic acid distinguishes Cannabis sativa samples from different plant species", FORENSIC SCIENCE INTERNATIONAL, vol. 106, no. 3, 1 December 1999 (1999-12-01), pages 135-146, XP55038622, ISSN: 0379-0738, DOI: 10.1016/S0379-0738(99)00193-0**
- **SALAMONE SALVATORE J ET AL: "A NON-CANNABINOID IMMUNOGEN USED TO ELICIT ANTIBODIES WITH BROAD CROSS-REACTIVITY TO CANNABINOID METABOLITES", JOURNAL OF FORENSIC SCIENCES, CALLAGHAN AND CO, CHICAGO, IL, US, vol. 43, no. 4, 1 January 1998 (1998-01-01), pages 821-826, XP008070042, ISSN: 0022-1198**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- H TANAKA: "Immunochemical Approach Using Monoclonal Antibody against delta9-Tetrahydrocannabinolic Acid (THCA) to Discern Cannabis Plants and to Investigate New Drug Candidates", CURRENT DRUG DISCOVERY TECHNOLOGIES, vol. 8, 1 January 2011 (2011-01-01), pages 3-15, XP55038676,
- SEBASTIAN DRESEN ET AL: "Development and validation of a liquid chromatography-tandem mass spectrometry method for the quantitation of synthetic cannabinoids of the aminoalkylindole type and methanandamide in serum and its application to forensic samples", JOURNAL OF MASS SPECTROMETRY, vol. 46, no. 2, 1 February 2011 (2011-02-01), pages 163-171, XP55032002, ISSN: 1076-5174, DOI: 10.1002/jms.1877
- Barry K Logan ET AL: "Technical Bulletin: NMS Labs test for JWH-018, JWH-019, JWH-073, JWH-250 and AM-2201 Primary Monohydroxy Metabolites in Human Urine", NMS LABS, 1 January 2011 (2011-01-01), XP55038739, Retrieved from the Internet: URL:http://toxwiki.wikispaces.com/file/view/JWH_metabolites_II_Technical_Bulletin_vers_1.1.pdf [retrieved on 2012-09-20]
- BARRY K. LOGAN ET AL: "Identification of Synthetic Cannabinoids in Herbal Incense Blends in the United States", JOURNAL OF FORENSIC SCIENCES, vol. 57, no. 5, 26 July 2012 (2012-07-26), pages 1168-1180, XP55038753, ISSN: 0022-1198, DOI: 10.1111/j.1556-4029.2012.02207.x

## Description

Background

The increasing rise in the use of stealth drugs (novel synthetic drugs that were previously or remain analytically/structurally uncharacterised and unclassified by government institutions), is exemplified by synthetic cannabinoid products which incorporate CP 47,497 as the active ingredient. Stealth synthetic cannabinoid (SSC) drug manufacturers can base their choice of active molecular target on scientific literature studies that address the therapeutic potential of $CB_1$ (the CNS cannabinoid receptor) agonists and antagonists. By incorporating novel, analytically uncharacterised compounds with high $CB_1$ receptor affinity into herbal mixtures (packaged under such names as Spice, Yucatan Fire), the manufacturers are able to target drug consumers clandestinely by promoting the material as herbal therapeutics. A problem for governments and drug enforcement agencies is that, even after identifying and banning a new synthetic cannabinoid, the manufacturers can rapidly react to the banning by incorporating a different active analogue into the same or a different herbal product; targeted minor changes in the molecular structure of the known active compound can preserve receptor activity but often produces a molecule whose GC-MS/LC-MS (the commonly applied detection techniques) profile is completely different from the original active molecule. Hence, the new active molecule initially remains unidentified and a further resource intensive and costly chemical analytical study to enable structural characterisation is required. The main active ingredients highlighted in SSC products to date are JWH-018, CP 47,497 and JWH-073 (Uchiyama et al. 2010; Hudson et al. 2010; Dresen et al. 2010). Initial studies of the metabolism of CP compounds have highlighted metabolic processes similar to tetrahydrocannabinol (THC) metabolism, namely ring and alkyl substituent hydroxylation, carboxylation and glucuronidation. As described herein, unless otherwise stated, CP refers to synthetic cannabinoid molecules comprising the unfused bicyclic structure II, in which X is either ethyl, *n*-propyl or *n*-butyl, as well as, metabolites thereof.

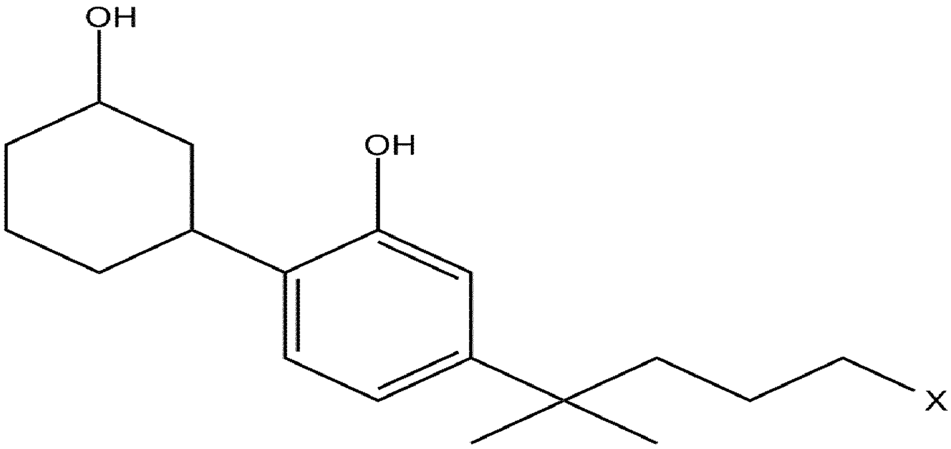

Structure II

Herbal therapeutics have been analysed using solvent extraction, pre-derivatisation and finally GC-MS analysis in SIM mode (Rana et al. 2010). This method is inadequate for the detection of future and 'current' JWH and CP SSCs (it is conceivable that 'current' herbal therapeutics, as well as CP 47,497, incorporate CP SSCs that are not yet characterised), requires sample pre-derivatisation, specialist staff for its implementation and expensive equipment. In Logan et al (2012) Journal of Forensic Sciences 57(5):1168-80, the identification of synthetic cannabinoids in herbal incense blends in the US using thin layer chromatography, gas chromatography mass spectrometry, high-performance liquid chromatography, and liquid chromatography time of flight mass spectrometry is reported upon.

In order to address the problem associated with the cheap and rapid detection of known CP molecules and their metabolites and/or future and associated metabolites based on the CP drug families, the Inventors devised a novel method based on novel antibodies raised from novel immunogens. The antibodies underpin an effective analytical and economic solution to the detection and quantification of current and future CP $CB_1$-active molecules in *in vitro* patient samples and herbal therapeutics.

References

Dresen et al. (2010). J. Mass Spectrom., 45: 1186-1194.
Hudson et al. (2010). J. Anal. Toxicol., 34: 252-260.
Rana et al. (2010). Quantitative composition of synthetic cannabinomimetics in "Herbal High" products. Poster at SOFT 2010 Annual Meeting, Richmond, Virginia, US.
Uchiyama et al. (2010). Foren. Sci. Int., 198: 31-38.

Summary of the Invention

The invention provides an antibody which binds to structure

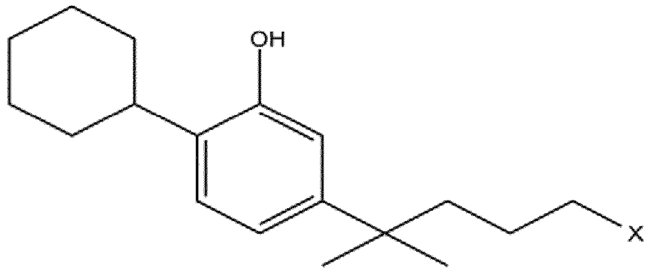

wherein: X is selected from *n*-propyl and *n*-butyl;
the antibody having a cross-reactivity represented by $B/B_0$ of ≤ 50% for an epitope selected from the group consisting of a racemic mixture of a $C_7$ homologue of CP-47,497 and a racemic mixture of a $C_8$ homologue of CP-47,497, at a cross-reactant concentration of 100 ng/ml; standardised with a racemic mixture of a $C_8$ homologue of CP-47,497 and using tracer **1** ($C_7$); the antibody further having a cross-reactivity represented by $B/B_0$ of > 80% for an epitope selected from the group consisting of a $C_7$ *para* quinone analogue of a racemic mixture of CP-47,497, (+-)-CP 55,940, (-)-CP 55,940, (+)-CP 55,940, HU-210, and HU-211 (Dexanabinol), at a cross-reactant concentration of 100 ng/ml; standardised with a racemic mixture of a $C_8$ homologue of CP-47,497 and using tracer **1** ($C_7$); wherein the cross-reactivity is determined using an Enzyme Linked Immunosorbent Assay (ELISA) technique as described in example 12, where B/B0 is determined by measuring absorbance at 450nm, where B is the absorbance at 450nm at x ng/ml standard concentration and where B0 is the absorbance at 450nm at 0 ng/ml standard concentration;
wherein the $C_7$ and $C_8$ homologues of CP-47,497 are:

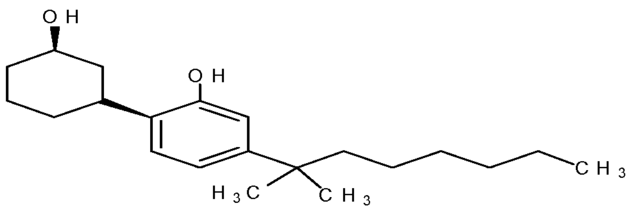

CP47,497 ($C_7$)

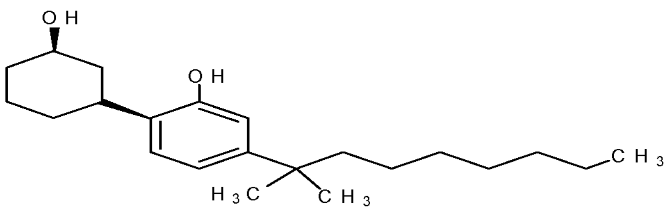

CP47,497 ($C_8$)

;

and
wherein tracer 1 is:

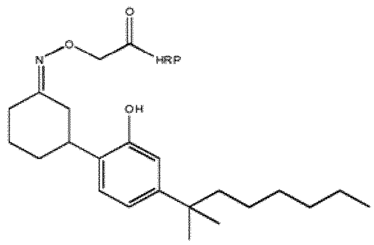

.

In an embodiment of the antibody of the invention, the antibody is raisable against an immunogen of structure:

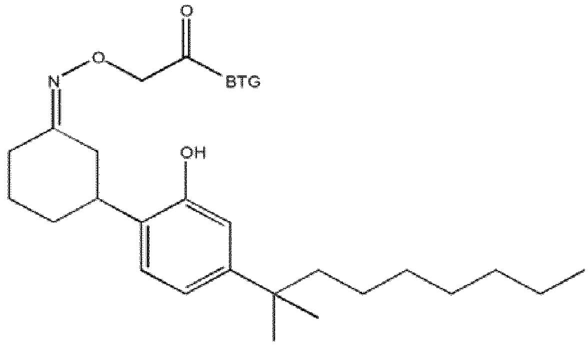

,

in which BTG is bovine thyroglobulin.

The invention also provides a method of detecting or determining synthetic cannabinoids of the CP family and/or one or more metabolites thereof in an *in vitro* sample of an individual or in a solution derived from a substance suspected of containing synthetic cannabinoids, comprising contacting the sample or solution with one or more detecting agents and one or more antibodies of the invention; detecting, or determining the quantity of, the one or more detecting agents; and deducing from calibrators, the presence of or amount of a molecule or molecules of the CP family and/or metabolites thereof in the sample or solution.

The invention further provides a kit for detecting or determining a molecule or molecules of the CP family and/or one or more metabolites thereof, comprising one or more antibodies of the invention.

The invention thus describes a rapid and practical method for the detection and determination of known and/or stealth synthetic cannabinoids based on the CP drug family. Kits and their use for CP SSC detection and determination in herbal therapeutics and *in vitro* patient samples are also described. The invention is underpinned by novel immunogens and antibodies which enable said methods, kits and applications.

Drawings

**Figure 1:** Chemical Structures of CP47,497 ($C_7$ & $C_8$). $C_7$ is used to denote a compound of structure II in which X is *n*-propyl and $C_8$ is used to denote a compound of structure II in which X is *n*-butyl.

**Figure 2**: Chemical Structures of CP47,497 ($C_7$ & $C_8$) Haptens.

**Figure 3a**: Chemical Reactions of Synthesis of CP47,497 ($C_7$) Hapten **1**.

**Figure 3b**: Chemical Reactions of Synthesis of CP47,497($C_8$) (Hapten **2**).

**Figure 4**: Chemical Structures of Immunogens **1b** and **2b** and of tracers **1** and **2**

**Figure 5:** Chemical Structures of (-)-CP 55940, (+)- CP55940 and CP 47497 para-quinone analogue

Detailed Description of the Invention

In a first aspect of the invention, there is provided an antibody which binds to an epitope of structure:

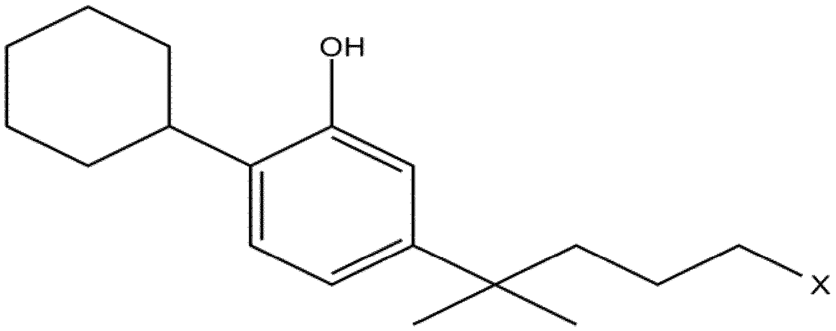

according to the claims, wherein X is selected from *n*-propyl and *n*-butyl. The definition of X is intended to embrace the CP-47,497 ($C_7$ homologue) and the CP-47,497 ($C_8$ homologue).

The antibody of the first aspect of the invention binds to an epitope selected from the group consisting of a racemic mixture of (±)-CP-47,497 ($C_7$ homologue), a racemic mixture of (±)-CP-47,497 ($C_8$ homologue), a stereoisomer of CP-47,497 ($C_7$ homologue) and a stereoisomer of CP-47,497 ($C_8$ homologue).

The antibody of first aspect of the invention is further characterised by having a $B/B_0$ of ≤ 50% for an epitope selected from the group consisting of a racemic mixture of a $C_7$ homologue of CP-47,497 and a racemic mixture of a $C_8$ homologue of CP-47,497, at a cross-reactant concentration of 100 ng/ml; standardised with a racemic mixture of a $C_8$ homologue of CP-47,497 and using tracer 1 ($C_7$); the antibody further having a cross-reactivity represented by $B/B_0$ of > 80% for an epitope selected from the group consisting of a $C_7$ *para* quinone analogue of a racemic mixture of CP-47,497, (+-)-CP 55,940, (-)-CP 55,940, (+)-CP 55,940, HU-210, and HU-211 (Dexanabinol), at a cross-reactant concentration of 100 ng/ml; standardised with a racemic mixture of a $C_8$ homologue of CP-47,497 and using tracer **1** ($C_7$).

The cross-reactivity is determined using an Enzyme Linked Immunosorbent Assay (ELISA) technique as described in example 12, where B/B0 is determined by measuring absorbance at 450nm, where B is the absorbance at 450nm at x ng/ml standard concentration and where B0 is the absorbance at 450nm at 0 ng/ml standard concentration, as explained herein.

The disclosure provides an antibody that is raisable against one or more immunogens of the following structures

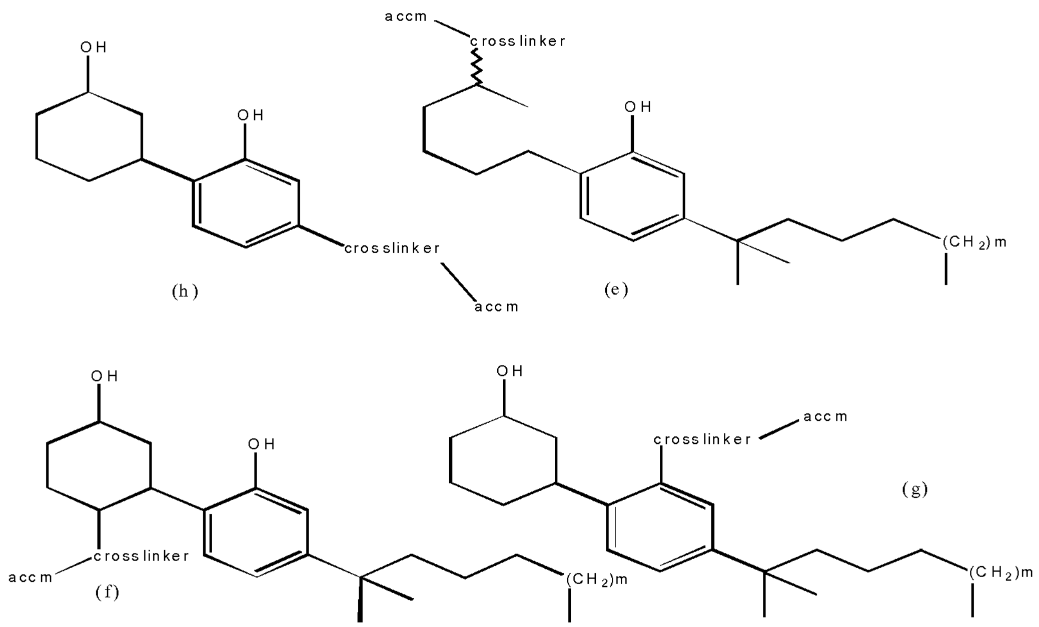

(h) (e) (f) (g)

Group II (e)-(h)

in which the accm is an antigenicity conferring carrier material; the crosslinker is a functionalised linking group joining the accm to the remainder of the molecule, the crosslinker of structure (e) forming either a single or double bond to the cyclohexyl ring and the crosslinker of structure (c) extending from the 4, 5, 6 or 7-position of the indole ring.

The antibody may be raisable against the immunogen of structure (e) and having a $B/B_0$ of ≤ 50% (standardised with (±)-CP-47,497 ($C_8$ homologue) and using tracer **1** ($C_7$).

An antibody of the disclosure may be raisable against the immunogen of structure (e) in which m=1-3, the crosslinker is either $-(L)_p$-M-Q- or =N-O-M-Q- in which Q, which is attached to the accm, is chosen from carbonyl, amino, thiol, maleimide, isocyanato, isothiocyanato, aldehyde, diazo and dithiopyridyl, M is a $C_{1-10}$ substituted or unsubstituted straight chain alkylene or arylene moiety, p=0 or 1 and L is O, NH, S, ester, thioester, or amide.

An antibody of the disclosure may be raisable against the immunogen of structure (e) in which m=1-2, the crosslinker is =N-O-M-Q- in which Q, which is attached to the accm, is chosen from carbonyl, amino, thiol, maleimide, isocyanato, isothiocyanato, aldehyde, diazo and dithiopyridyl and M is a $C_{1-10}$ substituted or unsubstituted straight chain alkylene or arylene moiety.

An antibody of the disclosure may be raisable against the immunogen of structure (e) in which the crosslinker is =N-O-M-Q- in which Q, which is attached to the accm, is carbonyl and M is a $C_1$ alkylene moiety.

An antibody of the disclosure may be raisable against the immunogen of structure (e), in which the immunogen of structure (e) is selected from the group consisting of

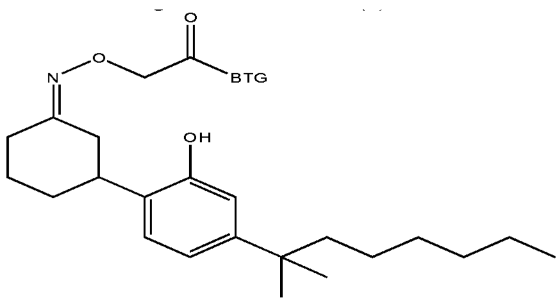

and

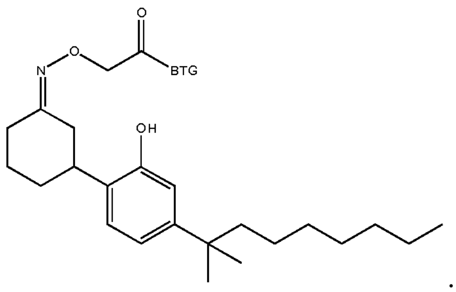

The antibody of the invention is raisable against the immunogen of structure (e), in which the immunogen is

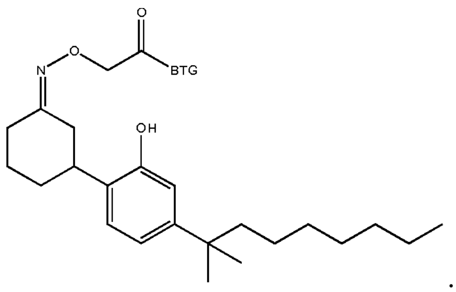

In a second aspect of the invention there is provided a method of detecting or determining synthetic cannabinoids of the CP family and/or one or more metabolites thereof in an *in vitro* sample of an individual or in a solution derived from a substance suspected of containing synthetic cannabinoids, comprising contacting the sample or solution with one or more detecting agents and one or more antibodies of the first aspect of the invention; detecting, or determining the quantity of, the one or more detecting agents; and deducing from calibrators, the presence of or amount of a molecule or molecules of the CP family and/or metabolites thereof in the sample or solution.

In a third aspect of the invention, there is provided a kit for detecting or determining a molecule or molecules of the CP family and/or one or more metabolites thereof, comprising one or more antibodies of the first aspect of the

invention.

The disclosure also provides one or more immunogens possessing the following structures

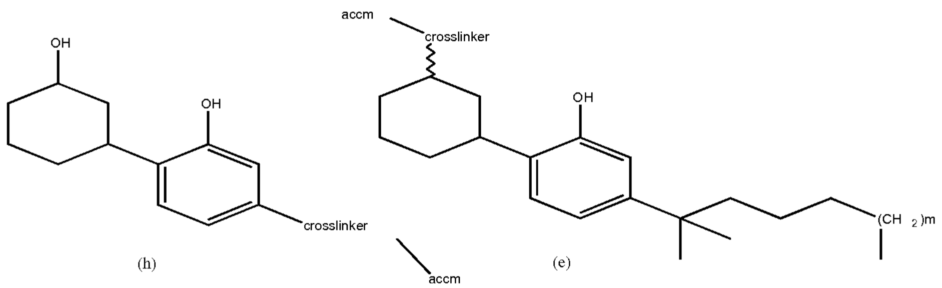

(h) (e)

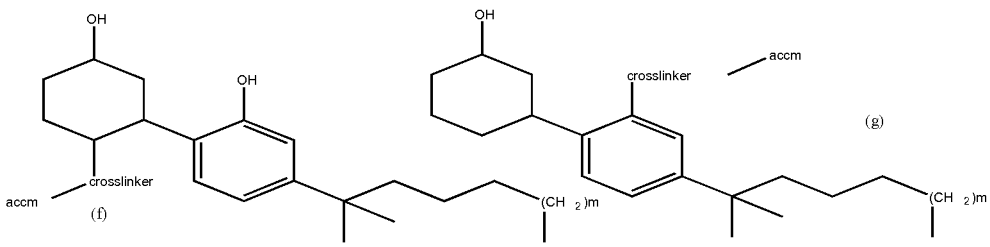

(f) (g)

Group II (e)-(h) (immunogens of the CP family)

in which the accm is an antigenicity conferring carrier material; m is 1 - 3; and the crosslinker is a functionalised linking group joining the accm to the remainder of the molecule.

By "functionalised", it is meant the crosslinker incorporates atoms that enable it to bond to both the accm and the CP moiety, forming a bridging group. In structure (e) the crosslinker forms either a single or double bond to the cyclohexyl ring. The crosslinker concept is well known to the person skilled in immunogen synthesis. For the current invention, when conjugating the hapten to the accm to form the immunogen, the nature and length of the crosslinker follows standard methods in order to optimise hapten epitopic recognition by the antibody. This entails a crosslinker of low immunogenicity and a chain length preferably of no greater than about ten atoms, most preferably no greater than six atoms.

Preferably for structures (f) and (h) the crosslinker is $-(A)_n$-D- Y- where A= O, -N(R)-, S, -S(O)-(sulphoxide) or $-S(O)_2$- (sulphonyl) and R=H or $C_1$-$_5$ alkyl, n= 0 or 1 and D is a $C_1$-$_{10}$, preferably a $C_{1-5}$ substituted or unsubstituted straight chain alkylene or arylene moiety and Y, which is attached to the accm, is selected from groups such as carbonyl, amino, thiol, maleimide, isocyanato, isothiocyanato, aldehyde, diazo and dithiopyridyl. Y is preferably carbonyl or amino.

Preferably for structure (e) m=2 or 3, the crosslinker is either $-(L)_p$-M-Q- or =N-O-M-Q- in which Q, attached to the accm, is either carbonyl or amino, M is a $C_{1-10}$, preferably a $C_{1-5}$ substituted or unsubstituted straight chain alkylene or arylene moiety, p=0 or 1 and L is O, NH, S, ester, thioester, or amide. More preferably, for structure (e) m=2 or 3, the crosslinker is =N-O-M-Q-, in which the crosslinker comprises =N-O-$CH_3$-COOH before attachment to the accm and =N-O-$CH_3$-CO- after attachment to the accm by an amide link.

Preferably for structure (g) m= 2 or 3, the crosslinker is $-(L)_p$-M-N- in which N, attached to the accm, is either carbonyl or amino, M is a $C_{1-10}$, preferably a $C_{1-5}$ substituted or unsubstituted straight chain alkylene or arylene moiety, p=0 or 1 and L is O, NH, S, ester, thioester, or amide.

The skilled person is aware that, for these antibodies to recognize CP molecules, they must bind to particular structures or epitopes of the hapten (in this context the hapten being that part of the immunogen that is not the crosslinker or accm); the epitopes are often distinct groups incorporating functional groups.

Another feature of the disclosure is an antibody raisable against an immunogen of structure (e), (f), (g) or (h), the antibody being able to bind to molecules of the CP family, metabolites of CP molecules and future SSC molecules comprising structure II. Optionally, the antibody is raisable against an immunogen of structure (e), the antibody being able to bind to molecules of the CP family, metabolites of CP molecules and future SSC molecules comprising structure II. When used in reference to an antibody, the word specific in the context of the current invention refers to the analyte that is preferably bound by the antibody, as gauged by a suitable metric such as the $IC_{50}$. Given the $IC_{50}$ of various analytes their cross-reactivities can be calculated.

The antibody of the invention can either be a polyclonal or monoclonal antibody using well-known methods. If the polyclonal antibody possesses the required specificity and sensitivity, that is, it binds a single analyte within the

detection range of the assay, development of a monoclonal antibody is unnecessary.

One or more antibodies of the invention can be incorporated into a kit for the detection and semi-determination of individual or multiple SSCs. The skilled person in the immunodiagnostic field is aware of several alternative immunoassay formats that could incorporate the antibodies of the invention either in solution or tethered (e.g. covalently bonded or electrostatically 'non-bonded' through Van der Waal's forces) to a solid substrate such as beads, glass/plastic slides or ceramic chips (a chip defined as a small, planar substrate). A preferred solid substrate onto which the antibodies of the invention are covalently bonded is a chip, preferably a ceramic chip; the word 'biochip'™ can be used to refer to a chip with antibodies attached. Such a "biochip" is described in EP1273349. Thus the invention also provides a solid substrate, preferably a biochip, comprising antibodies according to the invention.

The detection and determination criteria for a SSC using an immunoassay platform includes, as is well-known in the art, exceeding a pre-defined cut-off/concentration value or measuring the calibrator equivalent value as derived from a calibrator curve (also referred to as a standard curve).

Classification of immunoassays depends on whether one (noncompetitive) or two (competitive) antigens are used:

1. Competitive, homogeneous immunoassay

The antigen in the unknown sample competes with labeled antigen to bind with antibodies. The amount of unbound, labeled antigen is then measured, which is directly proportional to the concentration of sample antigen.

2. Competitive, heterogeneous immunoassay

The antigen in the unknown sample competes with labeled antigen to bind with antibodies. The amount of labeled antigen bound to the antibody site is then measured. In this method, the response will be inversely related to the concentration of antigen in the unknown.

3. One-site, noncompetitive immunoassay

The unknown antigen in the sample binds with labeled antibodies. The unbound, labeled antibodies are washed away, and the bound, labeled is measured, which is directly proportional to the amount of unknown antigen.

4. Two-site, noncompetitive immunoassays

The antigen in the unknown sample is bound to the antibody site, then labeled antibody is bound to the antigen. The amount of labeled antibody on the site is then measured. It will be directly proportional to the concentration of the antigen because labeled antibody will not bind if the antigen is not present in the unknown sample. This type is also known as sandwich assay as the antigen is "sandwiched" between two antibodies.

Another aspect of the invention is a method of detecting or determining synthetic cannabinoids of the CP family and their metabolites in an *in vitro* sample of an individual or in a solution derived from a substance suspected of containing synthetic cannabinoids comprising: contacting the sample or solution with one or more detecting agents and one or more antibodies of the invention that bind to molecules of the CP family, and detecting or determining, by reference to calibrators, the presence or concentration of a molecule or molecules of the CP family. Optionally, the method further comprises a step of measuring the detecting agents before detecting or determining, by reference to calibrators, the presence or concentration of a molecule or molecules of the CP family. The detecting agents measuring step can be measuring detecting agent bound to the antibodies, the detecting agent being labeled antigen in a competitive, homogeneous immunoassay in which unbound, labeled antigen is measured. Alternatively, the detecting agents measuring step can be measuring detecting agent bound to the antibodies, the detecting agent being labeled antigen in a competitive, heterogeneous immunoassay in which bound, labeled antigen is measured. Further alternatively, the detecting agents measuring step can be measuring detecting agent bound to the antibodies, the detecting agent being labeled antibodies in a one-site, noncompetitive immunoassay in which bound, labeled antibodies are measured. Still further alternatively, the detecting agents measuring step can be measuring detecting agent bound to the antibodies, the detecting agent being labeled antibody that is bound to the antigen that is, in turn, also bound to the antibody.

With reference to 'detecting or determining', 'detecting' means qualitatively analyzing for the presence or absence of a substance, 'determining' means quantitatively analyzing for the amount of a substance.

Optionally, the detecting agent is a small molecule, generally of similar structure to a molecule to be detected conjugated to a labelling agent, the detecting agent being able to bind to one of the antibodies of the invention.

The labelling agent is selected from an enzyme, a luminescent substance, a radioactive substance, or a mixture thereof. Preferably, the labelling agent is an enzyme, more preferably a peroxidase, most preferably horseradish peroxidase (HRP). Alternatively, or additionally, the luminescent substance may be a bioluminescent, chemiluminescent or fluorescent material.

For the purposes of the invention, the patient sample to be used for *in vitro* analysis can be hair or a peripheral biological fluid but is preferably whole blood, serum, plasma, or urine.

When referring to the detection or determination of a CP molecule, with or without a suffixed number attached to CP, the metabolite or metabolites are also inferred unless otherwise stated. The invention also describes kits for detecting or determining a molecule or molecules of the CP family comprising one or more antibodies of the invention. The kit comprises one or more antibodies raisable against an immunogen of structure (e) of Group II.

The antibodies of the kit are preferably tethered to any suitable solid support such as a chip. Although the solid support can be of any suitable shape such as a bead or a slide and of any suitable material such as silicon, glass or plastic, the solid support is preferably a ceramic chip.

The kit may further include calibrators and one or more detecting agents and optionally includes instructions for the use of the antibodies of the kit and if incorporated, the calibrators and detecting agents, for detecting and determining molecules from the CP family.

The invention also embodies solid supports comprising the novel antibodies of the present invention.

The antibodies of the invention are used for the detection or determination of CP molecules either in herbal mixtures, an *in vitro* sample taken from an individual or any other substance suspected of their incorporation.

## General Methods, Examples and Results

### *Preparation of Haptens, Immunogens and Detecting Agents*

Although haptens provide defined structural epitopes, they are not in themselves immunogenic and therefore need to be conjugated to carrier materials, which will elicit an immunogenic response when administered to a host animal. Appropriate carrier materials commonly contain poly(amino acid) segments and include polypeptides, proteins and protein fragments. Illustrative examples of useful carrier materials are bovine serum albumin (BSA), egg ovalbumin, bovine gamma globulin, bovine thyroglobulin (BTG), keyhole limpet haemocyanin (KLH) etc. Alternatively, synthetic poly(amino acids) having a sufficient number of available amino groups, such as lysine, may be employed, as may other synthetic or natural polymeric materials bearing reactive functional groups. Also, carbohydrates, yeasts or polysaccharides may be conjugated to the hapten to produce an immunogen.

The haptens can also be coupled to a detectable labelling agent such as an enzyme (for example, horseradish peroxidase), a substance having fluorescent properties or a radioactive label for the preparation of detecting agents for use in the immunoassays. The fluorescent substance may be, for example, a monovalent residue of fluorescein or a derivative thereof.

Immunogen formation for the invention described herein involves conventional conjugation chemistry. In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS).

General Procedure for MALDI-TOF Analysis of Immunogens.

MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1μl) were analysed using a matrix of sinapinic acid and bovine serum albumin (Fluka) was used as an external calibrant.

## Immunoassay Development

The process of developing an immunoassay is well known to the person skilled in the art. Briefly, for a competitive immunoassay in which the target analyte is a non-immunogenic molecule such as a hapten, the following process is conducted: antibodies are produced by immunising an animal, preferably a mammalian animal, by repeated administration of an immunogen. The serum from the immunised animal is collected when the antibody titre is sufficiently high. A detecting agent is added to a sample containing the target analyte and the raised antibodies, and the detecting agent and analyte compete for binding to the antibodies. The process may comprise fixing said serum antibodies to a backing substrate such as a polystyrene solid support or a ceramic chip. The antibodies can be polyclonal or monoclonal using standard techniques. The signal emitted in the immunoassay is proportionate to the amount of detecting agent bound to the antibodies which in turn is inversely proportionate to the analyte concentration. The signal can be detected or quantified by comparison with a calibrator.

Examples

Preparation of **Hapten 1** (see Figure 3a)

**Example 1:** Preparation of the Keto-CP47,497 ($C_7$) **1**

CP47,497 ($C_7$) (CAS 70434-82-1) is supplied as a 10mg/ml solution in methanol. First, it is transferred into 10ml round bottom flask (165ml, 165mg, 0.518mmol) and the solvent evaporated to dryness under vacuum at room temperature. Anhydrous dichloromethane (DCM) (5ml) is added and the resulting solution is added drop-wise to a solution of pyridinium chlorochromate (PCC) (167.6mg, 0.78mmol) in anhydrous dichloromethane (5ml) under stirring at room temperature (RT). The reaction mixture was stirred at room temperature for 3 hours. The reaction mixture was filtered on a pad of Celite™, washed with diethyl ether and the solvents were removed *in vacuo* at room temperature. The residue was dissolved in diethyl ether and taken through a small plug of silica gel, washed with diethyl ether and the solvents were removed *in vacuo* at room temperature to give the desired product, keto-CP47,497 ($C_7$) 1 (132mg, 0.417mmol, 80% yield) as a dark oil.

**Example 2:** Preparation of the CP47, 497 ($C_7$)-CMO (CP47,497 ($C_7$) carboxymethyloxime) **(Hapten 1)**

Keto-CP47,497 ($C_7$) **1** (132mg, 0.417mmol) was dissolved in pyridine (2ml) and carboxymethoxylamine hemihydrochloride (CMO.$^1/_2$HCl) (144mg, 1.04mmol, 2.5eq) was added. The reaction mixture was stirred at room temperature overnight. The solvents were evaporated, the residue was partitioned between ethyl acetate and HCl (1M) solution, the layers were separated and the organic layer was washed with brine, dried over sodium sulphate and concentrated *in vacuo* to give the desired product CP47,497 ($C_7$)-CMO **(Hapten 1)** (209mg) as a red-brown oil that was used in the next step without further purification.

**Example 3:** Conjugation of CP47,497 ($C_7$)-CMO **(Hapten 1)** to BSA - **Immunogen 1a**

To a solution of CP47,497 ($C_7$)-CMO **(Hapten 1)** (15.00mg, 0.0365mM) in DMF (2.0ml) was added EDC hydrochloride (8.85mg, 0.0462mM) and N-hydroxysuccinimide (5.4mg, 0.0462mM) and the mixture was stirred at room temperature overnight. This solution was added drop-wise to a solution of BSA (50.0mg, 1.15μM) in 50mM sodium bicarbonate solution (pH 8.5) (8.0ml). The mixture was then stirred overnight at 4°C. The solution was then dialysed against 50mM phosphate buffer pH 7.2 (3 changes) for 24 hours at 4°C, and freeze-dried to give 43mg of **Immunogen 1a.**

MALDI results showed 9.5 molecules of **hapten 1** had been conjugated to one molecule of BSA.

**Example 4:** Conjugation of CP47,497 ($C_7$)-CMO **(Hapten 1)** to BTG - **Immunogen 1b (Figure 4)**

To a solution of CP47,497 ($C_7$)-CMO **(Hapten 1)** (30.0mg, 0.073mM) in DMF (2.0ml) was added EDC hydrochloride (17.7mg, 0.092mM) and N-hydroxysuccinimide (11.5mg, 0.092mM) and the mixture was stirred at room temperature overnight. The solution was added drop-wise to a solution of BTG (100mg) in 50mM sodium bicarbonate solution (pH 8.5) (10ml). The mixture was then stirred overnight at 4°C. The solution was then dialysed against 50mM phosphate buffer pH 7.2 (3 changes) for 24 hours at 4°C, and freeze-dried to give 82mg of **Immunogen 1b.**

**Example 5:** Conjugation of CP47,497 ($C_7$)-CMO **(Hapten 1)** to HRP (horse radish peroxidase) **(Tracer 1) (Figure 4)**

EDC hydrochloride (10mg) was dissolved in water (0.5ml) and immediately added to a solution of CP47,497 ($C_7$)-CMO **(Hapten 1)** (2mg) in DMF (0.2ml). After mixing, this solution was added drop wise to a solution of HRP (20mg) in water (1ml). Sulfo-NHS (5mg) was added and the reaction mixture was incubated in the dark at room temperature overnight. Excess hapten was removed with double PD-10 columns (Pharmacia™) in series, pre-equilibrated with PBS at pH 7.2. The hapten-HRP conjugate was then dialysed overnight against 10L of PBS at pH 7.2 at 4°C.

Preparation of **Hapten 2** (see Figure 3b)

**Example 6:** Preparation of the Keto-CP47,497 ($C_8$) **2**

CP47,497 ($C_8$) (CAS 70434-92-3) is supplied as a 10mg/ml solution in methanol. First this solution was transferred into 10ml round bottom flask (194ml, 194mg, 0.583mmol) and the solvent evaporated to dryness under vacuum at room temperature. Anhydrous dichloromethane (5ml) is added and the resulting solution is added dropwise to a solution of pyridinium chlorochromate (188.6mg, 0.87mmol, 1.5eq) in anhydrous dichloromethane (5ml) under stirring

at room temperature. The reaction mixture was stirred at room temperature for 3 hours. The reaction mixture was filtered on a pad of Celite™, washed with diethyl ether and the solvents were removed *in vacuo* at room temperature. The residue was dissolved in diethyl ether and taken through a small plug of silica gel, washed with diethyl ether and the solvents were removed *in vacuo* at room temperature to give the desired product, keto-CP47,497 ($C_8$) 2 (148mg, 0.447mmol, 77% yield) as a dark oil.

**Example 7:** Preparation of the CP47,497 ($C_8$)-CMO **(Hapten 2)**

Keto-CP47,497 ($C_8$) **2** (148mg, 0.447mmol) was dissolved in pyridine (4ml) and CMO.$^1/_2$HCl (122mg, 1.12mmol) was added. The reaction mixture was stirred at room temperature overnight. The pyridine was removed under high vacuum. The obtained residue was partitioned between ethyl acetate and HCl (1M) solution, the layers were separated and the organic layer was washed with brine, dried over sodium sulphate and concentrated *in vacuo* to give the desired product CP47,497 ($C_8$)-CMO **(Hapten 2)** (200mg) as a red-brown oil that was used in the next step without further purification.

**Example 8:** Conjugation of CP47,497 ($C_8$)-CMO **(Hapten 2)** to BSA - **Immunogen 2a**

To a solution of CP47,497 ($C_8$)-CMO **(Hapten 2)** (15.5mg, 0.0384mM) in DMF (2.0ml) was added EDC hydrochloride (8.85mg, 0.0462mM) and N-hydroxysuccinimide (5.4mg, 0.0462mM) and the mixture was stirred at room temperature overnight. This solution was added drop-wise to a solution of BSA (50.0mg, 1.15μM) in 50mM sodium bicarbonate solution (pH 8.5) (8.0ml). The mixture was then stirred overnight at 4°C. The solution was then dialysed against 50mM phosphate buffer pH 7.2 (3 changes) for 24 hours at 4°C, and freeze-dried to give 40mg of **Immunogen 2a.**

MALDI results showed 16.03 molecules of CP47,497 ($C_8$)-CMO **(hapten 2)** had been conjugated to one molecule of BSA.

**Example 9:** Conjugation of CP47,497 ($C_8$)-CMO **(Hapten 1)** to BTG - **Immunogen 2b (Figure 4)**

To a solution of CP47,497 ($C_8$)-CMO **(Hapten 2)** (31.0mg, 0.073mM) in DMF (2.0ml) was added EDC hydrochloride (17.7mg, 0.092mM) and N-hydroxysuccinimide (11.5mg, 0.092mM) and the mixture was stirred at room temperature overnight. The solution was added drop-wise to a solution of BTG (100mg) in 50mM sodium bicarbonate solution (pH 8.5) (10ml). The mixture was then stirred overnight at 4°C. The solution was then dialysed against 50mM phosphate buffer pH 7.2 (3 changes) for 24 hours at 4°C, and freeze-dried to give 87mg of **Immunogen 2b.**

**Example 10:** Conjugation of CP47,497 ($C_8$)-CMO **(Hapten 2)** to HRP **(Tracer 2) (Figure 4)**

EDC hydrochloride (10mg) was dissolved in water (0.5ml) and immediately added to a solution of CP47,497 ($C_8$)-CMO **(Hapten 2)** (2mg) in DMF (0.2ml). After mixing, this solution was added drop wise to a solution of HRP (20mg) in water (1ml). Sulfo-NHS (5mg) was added and the reaction mixture was incubated in the dark at room temperature overnight. Excess hapten was removed with double PD-10 columns (Pharmacia™) in series, pre-equilibrated with PBS at pH 7.2. The hapten-HRP conjugate was then dialysed overnight against 10L of PBS at pH 7.2 at 4°C.

Preparation of Antisera

In order to generate polyclonal antisera, each of Immunogens **1b** and **2b** of the present invention is with Freund's Adjuvant and the mixture is injected into a host animal, such as rabbit, sheep, mouse, guinea pig or horse. Sheep are the preferred host animal. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification. However, in other cases, such as where the antibody is to be immobilised on a solid support, purification steps can be taken to remove undesired material and eliminate non-specific binding.

The specific antibodies prepared in this invention are useful as reagents in immunoassays for the detection or semi-determination of CP47,497 - $C_7$ and CP47,497 - $C_8$, in biological fluids.

**Example 11:** Preparation of antibodies to **Immunogen 1b** (CP47,497-$C_7$) and to **Immunogen 2b** (CP47,497 -$C_8$)

An aqueous solution of **Immunogen 1b** or **Immunogen 2b** was formulated with Freund's Complete Adjuvant (FCA) to form an emulsion consisting of 2mg/ml immunogen in 50% (v/v) FCA. Three sheep were immunised with this emulsion (1° immunisation), 0.25ml being intramuscularly injected at each of four sites in the flank of each animal.

Subsequent immunizations (boosts) contained 1mg/ml immunogen. All boosts were emulsified in 50% (v/v) Freund's Incomplete Adjuvant (FIA) and were administered in the same manner as the 1° immunisation, at monthly intervals. Blood sampling took place 7 to 14 days after each boost.

*Blood collection*

Briefly, blood is collected by applying pressure to the exposed jugular vein and inserting a clean 14 gauge hypodermic needle to remove 500ml of blood per sheep, under gravity. The blood is stored at 37°C for a minimum of 1 hour before the clots are separated from the side of the centrifuge bottles using disposable 1ml pipettes (ringing). The samples are stored at 4°C overnight.

*Processing & extraction of Immunoglobulin (Ig) fraction:*

Samples are centrifuged at 4000g for 30 minutes at 4°C. The serum is then poured off and centrifuged again at 16,000g for 15 minutes at 4°C, before being aliquoted and stored at <-20°C.

Precipitation of IgG from polyclonal antisera is carried out in two steps, using caprylic acid initially to precipitate most of the non-Ig G proteins, including albumin, followed by ammonium sulphate to extract IgG from the supernatant. This method produces a highly purified IgG fraction.

8ml of 60mM sodium acetate buffer, pH 4.4 is added to 2ml of antisera, followed by the addition of 200μl of caprylic acid. The resulting mixture is mixed on a roller for 30 minutes at room temperature. The precipitate is removed by centrifuging the samples at 1000g for 20 minutes at 4°C and filtering the supernatant through a 0.2μm Acrodisc™ filter. 1.4ml of 0.5M carbonate-bicarbonate buffer, pH 10.7, is added to each sample supernatant and cooled to 4°C. 9ml of saturated ammonium sulphate solution is added slowly whilst shaking and the resulting mixture is placed on a roller for 30 minutes at room temperature. The precipitate is extracted by centrifuging the samples at 1000g for 35 minutes at 4°C. The supernatant is poured off and the pellet re-suspended in 2ml PBS, pH 7.2. The sample is dialysed overnight at 4°C in PBS, pH7.2 containing 0.09% azide. After dialysis, the sample is filtered using a 0.2μm Acrodisc™ filter and aliquoted for storage at <20°C. The IgG fraction can then be evaluated by competitive ELISA microtiter plate assay, as described below.

**Example 12:** Characterisation of antibodies to **Immunogen 2b** (CP47,497 -$C_8$)

Standard curves

The wells of an enhanced binding 96 well polystyrene microtiter plate were coated with IgG fraction of antiserum (Antibody 4.1 or antibody 4.2) raised to Immunogen 4 in separate host animals, diluted in 10mM Tris, pH8.5 (125μl/well). The appropriate antibody coating dilution was determined using standard ELISA checkerboard techniques. The plate was incubated overnight at 4°C, washed 4 times over 10 minutes with Tris buffered saline containing Tween 20 (TBST) and tapped dry.

Standard solutions of CP47,497 -$C_8$ were prepared in TBST at 0, 2.5, 5, 10, 20, 40, 80 and 160ng/ml for Antibody 4.1, and at 0, 5, 10, 20, 40, 80, 160 and 320ng/ml for Antibody 4.2, and 50μl of each was added to the appropriate wells. 75μl of conjugate (**Hapten 1**-HRP - tracer **1 -** see Figure 4) diluted in Tris buffer (pH 7.2) containing EDTA, D-mannitol, sucrose, thimerosal and BSA, was added to each of the wells. The appropriate dilution of conjugate was also determined using standard ELISA checkerboard techniques. The plate was incubated at 25°C for 1 hour. Excess unbound conjugate was removed by washing 6 times over a 10 minute period with TBST. 125μl of tetramethylbenzidine (TMB) substrate solution was added to each well of the plate that was then incubated for 20 minutes in the dark at room temperature. The reaction was terminated by addition of 125μl 0.2M $H_2SO_4$ to each well. The absorbance was then measured at 450nm using a microtiter plate reader. The data generated in the assay is presented in **Table 1.**

**Table 1:** Data generated from competitive microtiter plate assays for CP47,497 -$C_8$, employing antisera raised against Immunogen **2b** ($C_8$) (antibody 4.1 in Table 1a and antibody 4.2 in Table 1b), tracer **1** ($C_7$) and, as standard, (±)-CP-47,497 ($C_8$ homologue).

Table 1a -

| CP47,497 ($C_8$) | Antibody 4.1 | |
|---|---|---|
| ng/ml | $A_{450}$ | %B/$B_0$ |
| 0 | 1.866 | 100 |
| 2.5 | 1.529 | 82 |

(continued)

| CP47,497 ($C_8$) | Antibody 4.1 | |
|---|---|---|
| ng/ml | $A_{450}$ | %B/$B_0$ |
| 5 | 1.339 | 72 |
| 10 | 1.117 | 60 |
| 20 | 0.945 | 51 |
| 40 | 0.808 | 43 |
| 80 | 0.700 | 38 |
| 160 | 0.641 | 34 |
| $IC_{50}$ (ng/ml) | 20.922 | |

Table 1b

| CP47,497 ($C_8$) | Antibody 4.2 | |
|---|---|---|
| ng/ml | $A_{450}$ | %B/$B_0$ |
| 0 | 1.877 | 100 |
| 5 | 1.657 | 88 |
| 10 | 1.546 | 82 |
| 20 | 1.356 | 72 |
| 40 | 1.184 | 63 |
| 80 | 0.979 | 52 |
| 160 | 0.827 | 44 |
| 320 | 0.698 | 37 |
| $IC_{50}$ (ng/ml) | 95.992 | |
| $A_{450}$ = absorbance at 450nm<br>B = absorbance at 450nm at xng/ml standard concentration<br>$B_0$ = absorbance at 450nm at 0ng/ml standard concentration<br>$IC_{50}$ = standard concentration which produces 50% B/B | | |

Antibody 4.2 shows a higher $IC_{50}$ than antibody 4.1. This is reflected in their respective titres (not shown). Antibody 4.2 may show a lower $IC_{50}$ when purified from subsequent immunisations from the same host animal. In the next experiment, each of antibodies 4.1 and 4.2 show a similar cross-reactivity pattern.

Cross reactivity

In order to determine the specificity of the competitive ELISAs, standard solutions of a range of structurally similar compounds were prepared in TBST. Using the calibration curves generated and employing a single level of cross reactants (100ng/ml), these were used to determine the cross-reactivity of each immunoassay with these substances. The results of this study are presented in **Table 2,** as %B/$B_0$, employing antisera raised against Immunogen **2b** ($C_8$), tracer **1** ($C_7$) and (±)-CP-47,497 ($C_8$ homologue) as standard.

**Table 2:**

| Cross-reactants at 100ng/ml | | Antibody 4.1 | Antibody 4.2 |
|---|---|---|---|
| | | %B/$B_0$ | %B/$B_0$ |
| 1 | (±)-CP-47,497 ($C_7$ homologue) | 37.14 | 48.85 |

(continued)

| Cross-reactants at 100ng/ml | | Antibody 4.1 | Antibody 4.2 |
|---|---|---|---|
| | | %B/$B_0$ | %B/$B_0$ |
| 2 | (±)-CP-47,497 ($C_7$ *para* quinone analogue), identified as "cayman 10899" | 82.66 | 89.82 |
| 3 | (±)-CP-47,497 ($C_8$ homologue) | 36.52 | 49.65 |
| 4 | ((+-)-CP 55,940) - CAS No. 83003-12-7 | 93.65 | 97.34 |
| 5 | (-)-CP 55,940 - CAS No. 83002-04-4 | 91.26 | 97.23 |
| 6 | (+)-CP 55,940, identified as "cayman 13608" | 95.98 | 96.91 |
| 7 | HU-210 - CAS No. 112830-95-2 | 96.01 | 92.94 |
| 8 | HU-211 (Dexanabinol) - CAS No. 112924-45-5 | 96.76 | 95.84 |
| 9 | HU-308 - CAS No. 256934-39-1 | 98.37 | 94.81 |
| 10 | Delta 9 THC - CAS No. 1972-08-3 | 96.44 | 94.09 |
| 11 | (-)-11-nor-9-Carboxy-delta9-THC - CAS No. 56354-06-4 | 95.23 | 94.38 |
| (+)- CP55940 and (-)- CP55940 are illustrated in Figure 5 and provided by Cayman Chemicals.<br>((+-)-CP 55,940) is a racemic mixture of (+)- CP55940 and (-)- CP55940.<br>(±)-CP-47,497 ($C_7$ *para* quinone analogue) is also illustrated in Figure 5. Each of (±)-CP-47,497 ($C_7$ homologue) and (±)-CP-47,497 ($C_8$ homologue) is a mixture of the 2 possible cyclohexyl cis configurations. | | | |

Cross-reactivity is calculated according to the following formula:

$$\%CR = IC_{50,\ CP47,\ 497\ (C8)} / IC_{50,\ CR} \ X\ 100$$

Where %CR is the percentage cross-reactivity, $IC_{50,\ CP47,\ 497\ (C8)}$ is the concentration of CP47, 497 ($C_8$) that causes 50% displacement of signal and $IC_{50,\ CR}$ is the concentration of CP synthetic cannabinoid/metabolite/selected molecule that causes 50% displacement of signal.

The cross-reactivity data show that antibodies raised against immunogen **2b** bind to each of (±)-CP-47,497 ($C_7$ homologue) and (±)-CP-47,497 ($C_8$ homologue) - these antibodies show a %B/$B_0$ of less than 50% with each of these cross-reactants, using tracer **1** ($C_7$) and (±)-CP-47,497 ($C_8$ homologue) as standard. A similar %B/$B_0$ of less than 50% is also expected for each stereoisomer in the racemic mixture forming each of (±)-CP-47,497 ($C_7$ homologue) and (±)-CP-47,497 ($C_8$ homologue). In contrast, antibodies raised against immunogen **2b** show a %B/$B_0$ of greater than 80% with each of ((+-)-CP 55,940), (-)-CP 55,940 and (+)-CP 55,940. It will be appreciated that, in CP-47,497, there is a hydroxyl substituent at position 3 of the cyclohexane ring and hydrogen substituents at the 6 position. In contrast, in CP-55,940, there remains a hydroxyl substituent at position 3 of the cyclohexane ring but one of the hydrogen substituents at the 6 position is replaced by a propyl group terminating in a hydroxyl group. It would appear that antibodies raised against immunogen **2b** require 2 hydrogen substituents at position 6 of the cyclyohexyl ring.

In (±)-CP-47,497 ($C_7$ *para* quinone analogue), the phenyl ring of (±)-CP-47,497 ($C_7$ homologue) is replaced by a *p*-benzoquinone ring. Antibodies raised against immunogen **2b** show a %B/$B_0$ of greater than 80% with (±)-CP-47,497 ($C_7$ *para* quinone analogue). It would appear that antibodies raised against immunogen **2b** require a hydrogen substituent at position 5 of the phenyl ring.

In HU-210 and HU-211, there is an additional ring bridging position 6 of the cyclohexyl ring and position 6 of the phenyl ring. Antibodies raised against immunogen **2b** show a %B/$B_0$ of greater than 80% with each of HU-210 and HU-211. It would appear that antibodies raised against immunogen **2b** require a hydrogen substituent at position 6 of the phenyl ring and 3 hydrogen substituents at position 6 of the cyclohexyl ring.

## Claims

**1.** An antibody which binds to structure

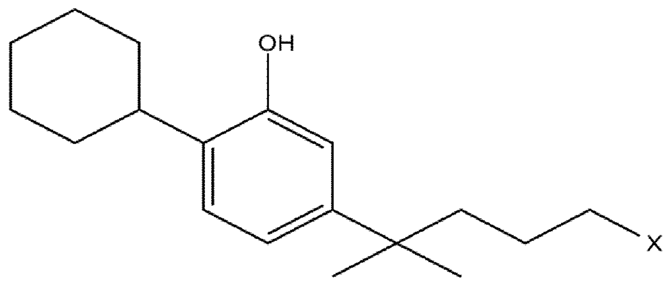

wherein:

X is selected from *n*-propyl and *n*-butyl;

the antibody having a cross-reactivity represented by $B/B_0$ of ≤ 50% for an epitope selected from the group consisting of a racemic mixture of a $C_7$ homologue of CP-47,497 and a racemic mixture of a $C_8$ homologue of CP-47,497, at a cross-reactant concentration of 100 ng/ml; standardised with a racemic mixture of a $C_8$ homologue of CP-47,497 and using tracer **1** ($C_7$);

the antibody further having a cross-reactivity represented by $B/B_0$ of > 80% for a $C_7$ *para* quinone analogue of a racemic mixture of CP-47,497, (+-)-CP 55,940, (-)-CP 55,940, (+)-CP 55,940, HU-210, and HU-211 (Dexanabinol), at a cross-reactant concentration of 100 ng/ml; standardised with a racemic mixture of a $C_8$ homologue of CP-47,497 and using tracer **1** ($C_7$);

wherein the cross-reactivity is determined using an Enzyme Linked Immunosorbent Assay (ELISA) technique as described in example 12, where $B/B_0$ is determined by measuring absorbance at 450nm, where B is the absorbance at 450nm at x ng/ml standard concentration and where $B_0$ is the absorbance at 450nm at 0 ng/ml standard concentration;

wherein the $C_7$ and $C_8$ homologues of CP-47,497 are:

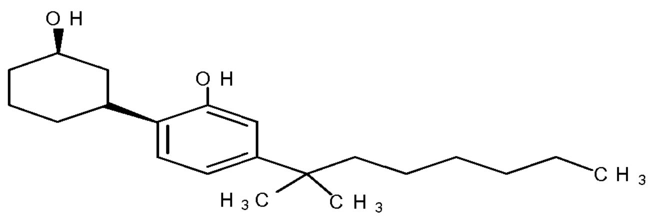

**CP47,497 ($C_7$)**

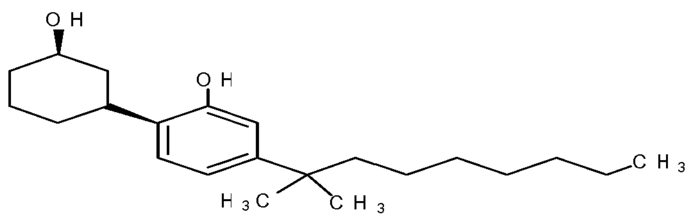

**CP47,497 ($C_8$)**

;

and

wherein tracer 1 is:

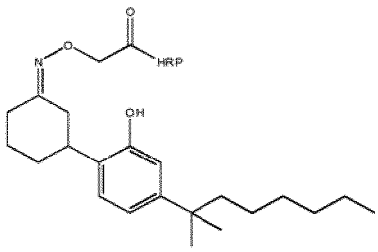

.

2. The antibody according to claim 1, wherein the antibody is raisable against an immunogen of structure:

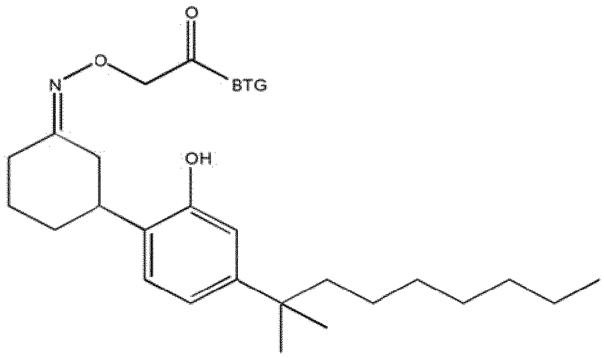

,

in which BTG is bovine thyroglobulin.

3. A method of detecting or determining synthetic cannabinoids of the CP family and/or one or more metabolites thereof in an *in vitro* sample of an individual or in a solution derived from a substance suspected of containing synthetic cannabinoids, comprising contacting the sample or solution with one or more detecting agents and one or more antibodies of Claim 1 or 2; detecting, or determining the quantity of, the one or more detecting agents; and deducing from calibrators, the presence of or amount of a molecule or molecules of the CP family and/or metabolites thereof in the sample or solution.

4. A kit for detecting or determining a molecule or molecules of the CP family and/or one or more metabolites thereof, comprising one or more antibodies of Claim 1 or 2.

**Patentansprüche**

1. Antikörper, der sich an die folgende Struktur bindet:

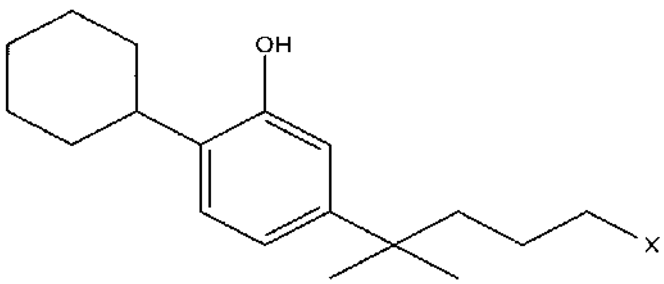

wobei:

X aus *n*-Propyl und *n*-Butyl ausgewählt ist;
der Antikörper eine Kreuzreaktivität, die durch $B/B_0$ von ≤ 50 % für ein Epitop dargestellt wird, das aus der Gruppe ausgewählt ist, die aus einer racemischen Mischung eines $C_7$-Homologs von CP-47,497 und einer racemischen Mischung eines $C_8$-Homologs von CP-47,497 besteht, bei einer Kreuzreaktionsmittelkonzentration

von 100 ng/ml, aufweist; die mit einer racemischen Mischung eines $C_8$-Homologs von CP-47,497 und unter Verwendung von Tracer **1** ($C_7$) standardisiert ist;
der Antikörper ferner eine Kreuzreaktivität, die durch $B/B_0$, von > 80 % für ein $C_7$-Para-Chinon-Analogon einer racemischen Mischung von CP-47,497, (+-)-CP 55,940, (-)-CP 55,940, (+)-CP 55,940, HU-210 und HU-211 (Dexanabinol) dargestellt wird, bei einer Kreuzreaktionsmittelkonzentration von 100 ng/ml, aufweist; die mit einer racemischen Mischung eines $C_8$-Homologs von CP-47,497 und unter Verwendung von Tracer **1** ($C_7$) standardisiert ist;
wobei die Kreuzreaktivität unter Verwendung einer Enzymimmunoassay(*Enzyme Linked Immunosorbent Assay*- ELISA)-Technik, wie in Beispiel 12 beschrieben, bestimmt wird, wobei $B/B_0$ durch Messen einer Absorption bei 450 nm bestimmt wird, wobei B die Absorption bei 450 nm bei *x* ng/ml Standardkonzentration ist und wobei $B_0$ die Absorption bei 450 nm bei 0 ng/ml Standardkonzentration ist;
wobei die $C_7$ - und $C_8$-Homologen von CP-47,497 Folgendes sind:

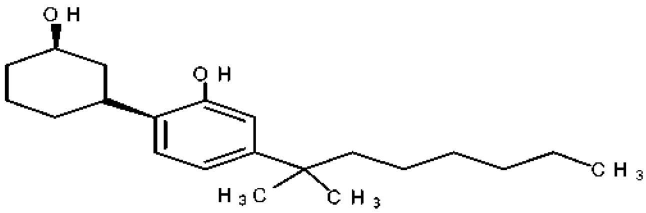

CP47,497 ($C_7$)

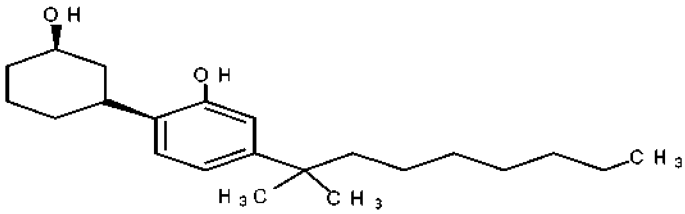

CP47,497 ($C_8$)

;

und
wobei Tracer 1 Folgendes ist:

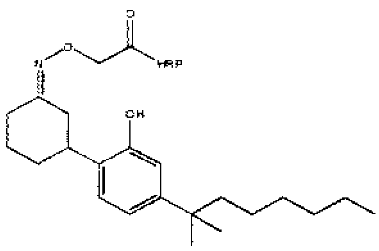

**2.** Antikörper nach Anspruch 1, wobei der Antikörper gegen ein Immunogen der folgenden Struktur anhebbar ist:

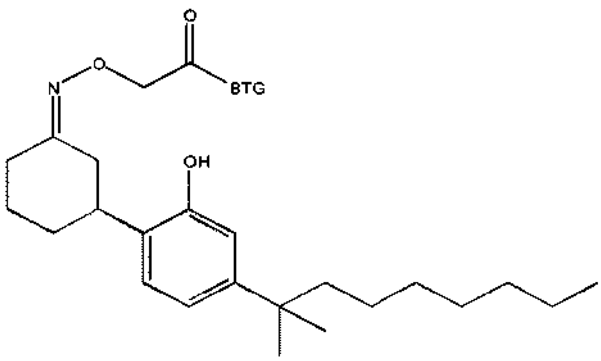

,

in der BTG bovines Thyreoglobulin ist.

3. Verfahren zum Nachweisen oder Bestimmen synthetischer Cannabinoide der CP-Familie und/oder eines oder mehrerer Metaboliten davon in einer *In-vitro*-Probe eines Individuums oder in einer Lösung, die von einer Substanz abstammt, von der vermutet wird, dass sie synthetische Cannabinoide enthält, die das Inberührungbringen der Probe oder Lösung mit einem oder mehreren Nachweismitteln und einem oder mehreren Antikörpern nach Anspruch 1 oder 2 umfasst; Nachweisen oder Bestimmen der Menge eines oder mehrerer Nachweismittel; und Ableiten des Vorhandenseins oder der Menge eines Moleküls oder von Molekülen der CP-Familie und/oder deren Metaboliten in der Probe oder Lösung aus Kalibratoren.

4. Kit zum Nachweisen oder Bestimmen eines Moleküls oder von Molekülen der CP-Familie und/oder eines oder mehrerer Metaboliten davon, das einen oder mehrere Antikörper nach Anspruch 1 oder 2 umfasst.

## Revendications

1. Anticorps qui se lie à la structure

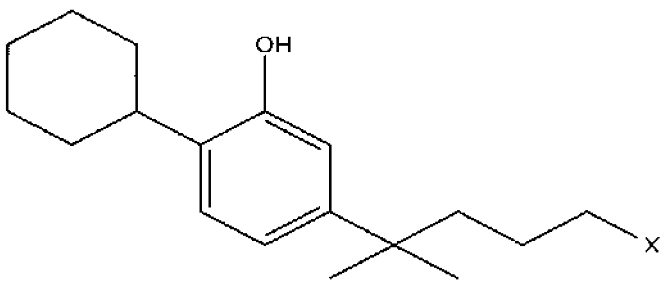

dans lequel :

X est choisi parmi le *n*-propyle et le *n*-butyle ;
l'anticorps présentant une réactivité croisée représentée par $B/B_0$ de ≤ 50 % pour un épitope choisi dans le groupe constitué par un mélange racémique d'un homologue $C_7$ de CP-47,497 et un mélange racémique d'un homologue $C_8$ de CP-47,497, à une concentration en réactif croisé de 100 ng/ml ; standardisé avec un mélange racémique d'un homologue $C_8$ de CP-47,497 et à l'aide du traceur 1 ($C_7$) ;
l'anticorps présentant en outre une réactivité croisée représentée par $B/B_0$ de > 80 % pour un analogue de para quinone $C_7$ d'un mélange racémique de CP-47,497, (+-)-CP 55,940, (-)-CP 55,940, (+)-CP 55,940, HU-210 et HU-211 (dexanabinol), à une concentration en réactif croisé de 100 ng/ml ; standardisé avec un mélange racémique d'un homologue $C_8$ de CP-47,497 et à l'aide du traceur 1 ($C_7$) ;
dans lequel la réactivité croisée est déterminée à l'aide d'une technique de dosage immuno-enzymatique (ELISA) telle que décrite dans l'exemple 12, où $B/B_0$ est déterminé par mesure de l'absorbance à 450 nm, où B représente l'absorbance à 450 nm à une concentration standard *x* ng/ml et où $B_0$ représente l'absorbance à 450 nm à une concentration standard de 0 ng/ml ;
dans lequel les homologues $C_7$ et $C_8$ de CP-47,497 sont :

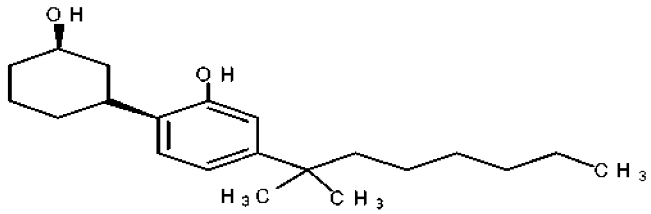

CP47,497 ($C_7$)

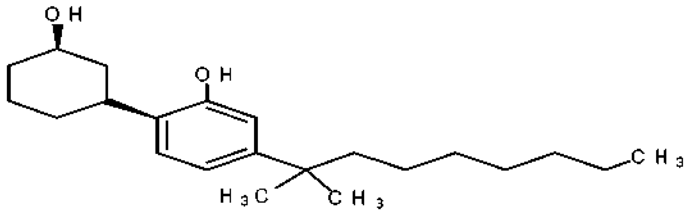

CP47,497 ($C_8$)

;

et
dans lequel le traceur 1 est :

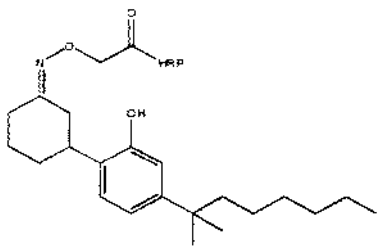

2. Anticorps selon la revendication 1, dans lequel l'anticorps peut être développé sur un immunogène de structure :

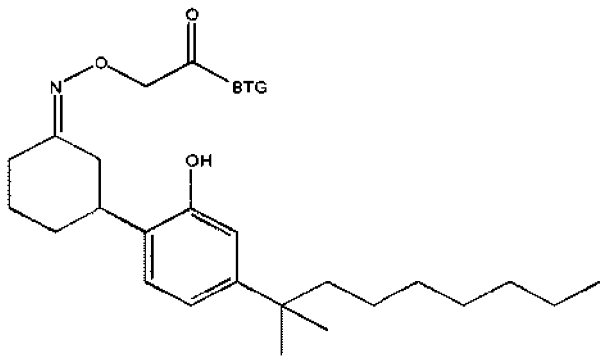

,

dans laquelle la BTG est la thyroglobuline bovine.

3. Procédé de détection ou de détermination de cannabinoïdes synthétiques de la famille CP et/ou d'un ou de plusieurs métabolites de ceux-ci dans un échantillon in vitro d'un individu ou dans une solution dérivée d'une substance suspectée de contenir des cannabinoïdes synthétiques, comprenant la mise en contact de l'échantillon ou de la solution avec un ou plusieurs agents détecteurs et un ou plusieurs anticorps selon la revendication 1 ou 2 ;
la détection ou la détermination de la quantité du ou des agents détecteurs ; et
la déduction à partir de calibrateurs de la présence ou quantité d'une molécule ou de molécules de la famille des CP et/ou de leurs métabolites dans l'échantillon ou la solution.

4. Kit pour détecter ou déterminer une molécule ou des molécules de la famille des CP et/ou un ou plusieurs de leurs métabolites, comprenant un ou plusieurs anticorps selon la revendication 1 ou 2.

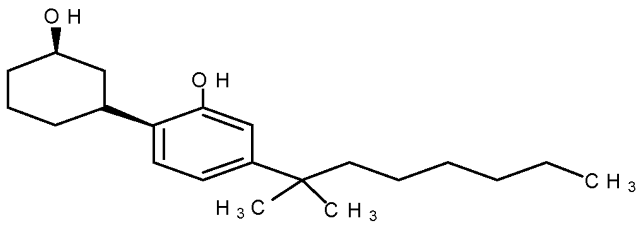

CP47, 497 ($C_7$)

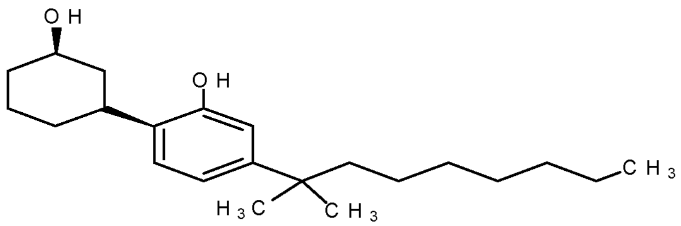

CP47, 497 ($C_8$)

**Figure 1**: Chemical Structures of CP47,497 ($C_7$ & $C_8$)

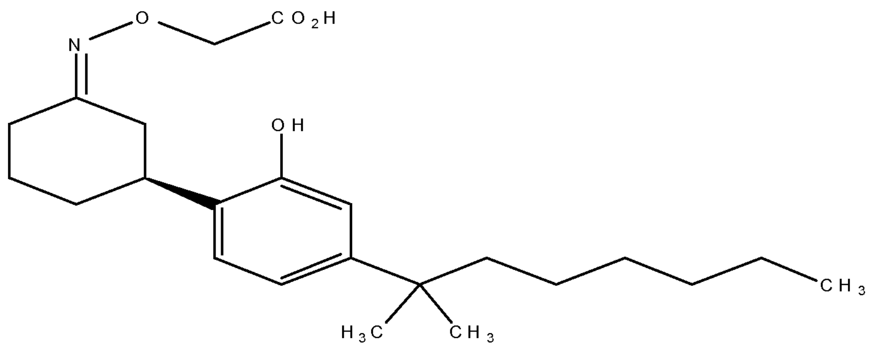

CP47, 497 ($C_7$) CMO (Hapten - 1)

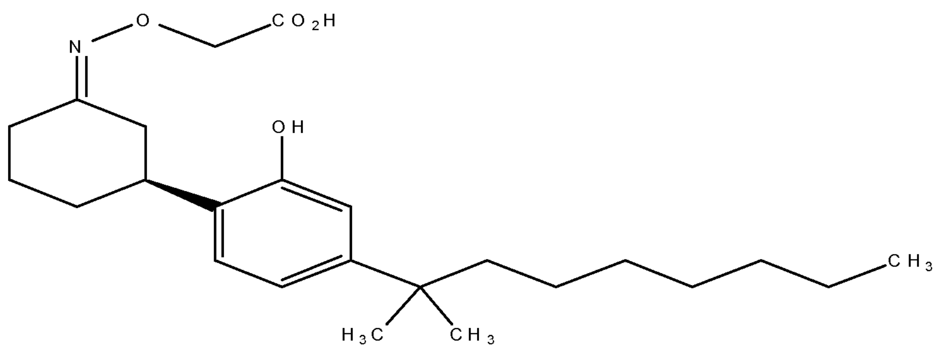

CP47, 497 ($C_8$) CMO (Hapten - 2)

**Figure 2**: Chemical Structures of CP47,497 ($C_7$ & $C_8$) Haptens 1 and 2

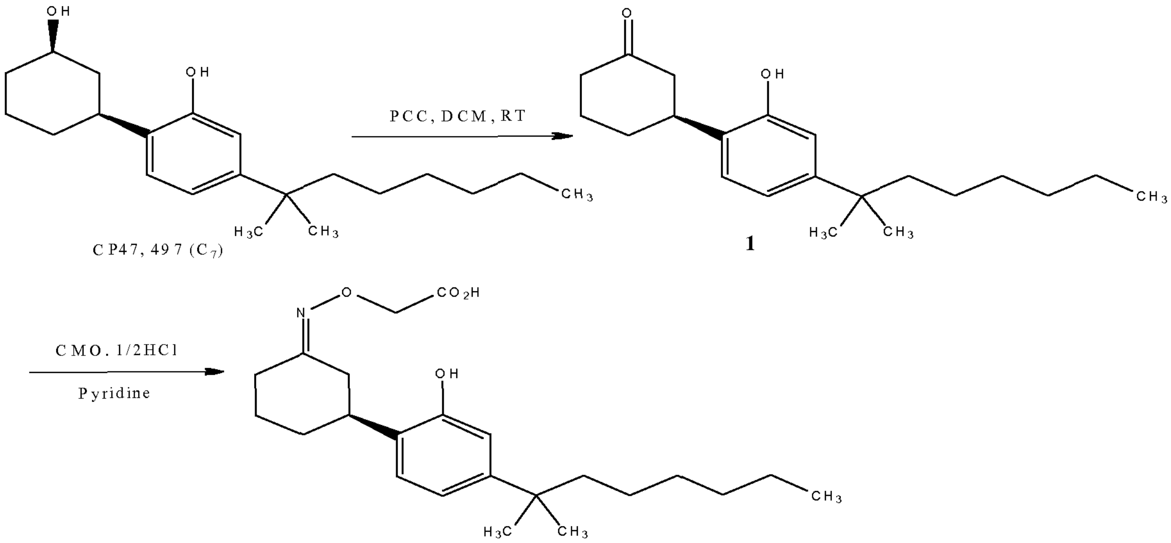


**Hapten - 1 [CP47, 497 ($C_7$) CMO]**

**Figure 3a**: Chemical Reactions of Synthesis of CP47, 497 ($C_7$) Hapten **1**

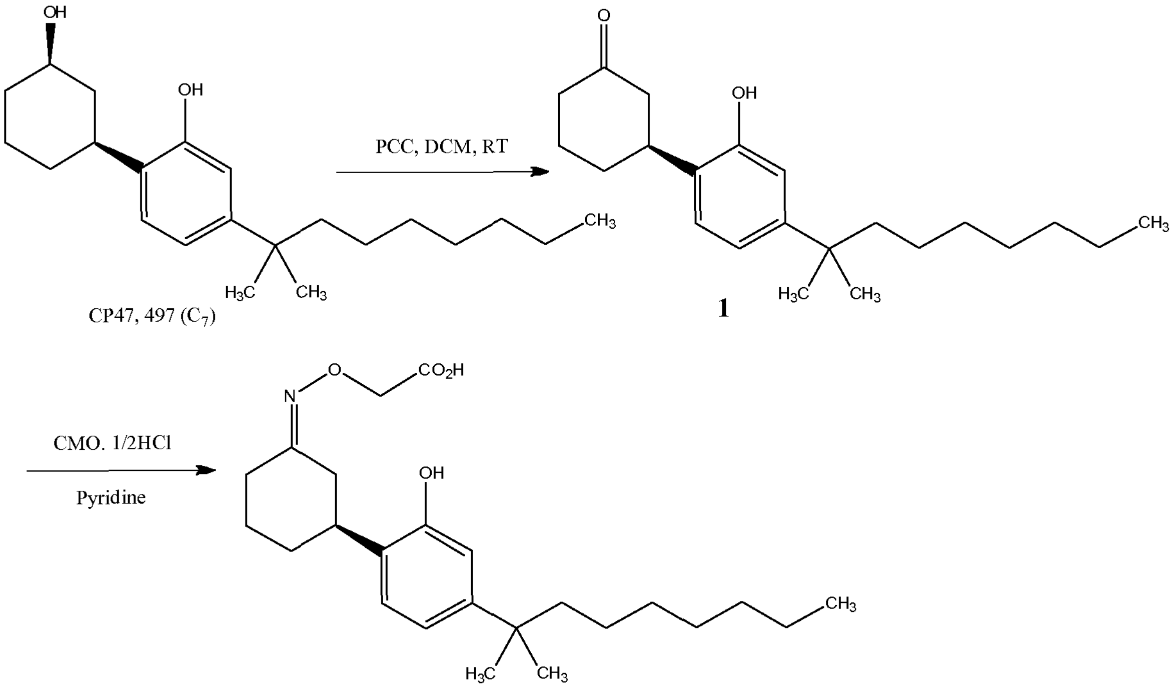


**Hapten - 1 [CP47, 497 ($C_7$) CMO]**

**Figure 3b**: Chemical Reactions of Synthesis of CP47, 497($C_8$) (Hapten **2**)

**Figure 4**

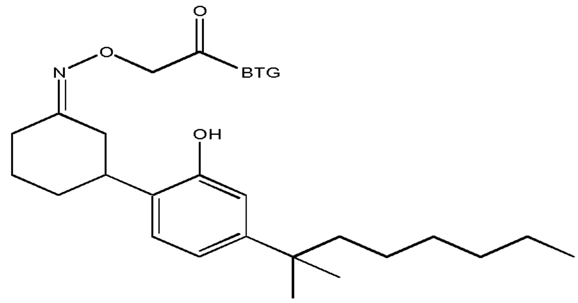

Immunogen **1b**

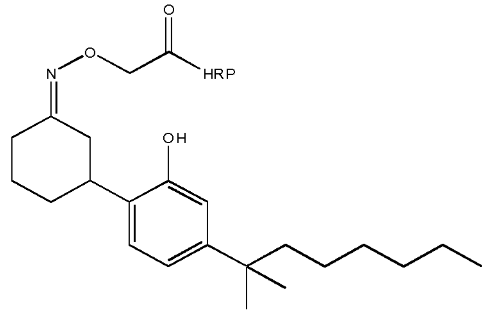

Tracer **1**

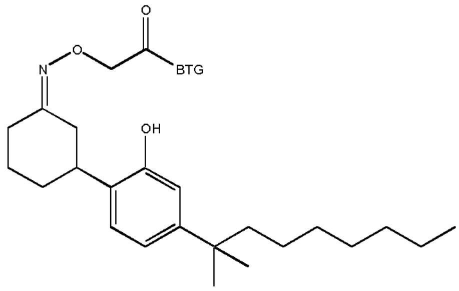

Immunogen **2b**

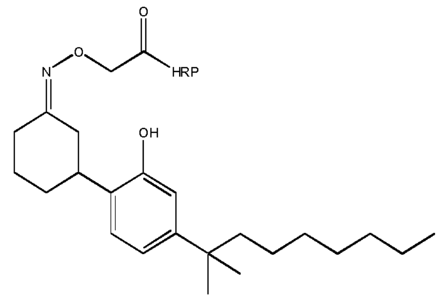

Tracer **2**

**Figure 5**

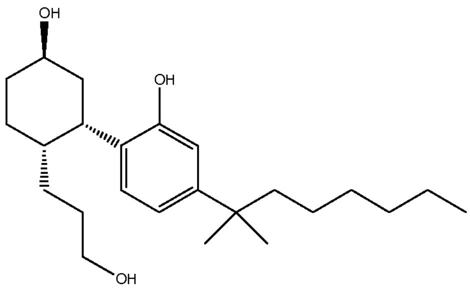

(-)-CP 55940

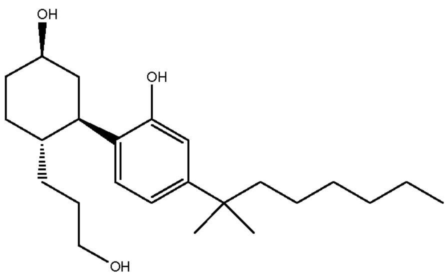

(+)- CP55940

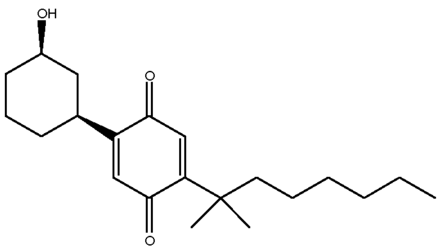

CP 47497 para-quinone analogue

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1273349 A


### Non-patent literature cited in the description


- **LOGAN et al.** *Journal of Forensic Sciences,* 2012, vol. 57 (5), 1168-80
- **DRESEN et al.** *J. Mass Spectrom.,* 2010, vol. 45, 1186-1194
- **HUDSON et al.** *J. Anal. Toxicol.,* 2010, vol. 34, 252-260
- **RANA et al.** Quantitative composition of synthetic cannabinomimetics. *Herbal High'' products. Poster at SOFT 2010 Annual Meeting,* 2010
- **UCHIYAMA et al.** *Foren. Sci. Int.,* 2010, vol. 198, 31-38
- *CHEMICAL ABSTRACTS,* 83003-12-7
- *CHEMICAL ABSTRACTS,* 83002-04-4
- *CHEMICAL ABSTRACTS,* 112830-95-2
- *CHEMICAL ABSTRACTS,* 112924-45-5
- *CHEMICAL ABSTRACTS,* 256934-39-1
- *CHEMICAL ABSTRACTS,* 1972-08-3
- *CHEMICAL ABSTRACTS,* 56354-06-4